# EUROPEAN PATENT SPECIFICATION

(11) **EP 2 191 023 B1**
(45) Date of publication and mention of the grant of the patent: **14.01.2015**
(21) Application number: 08805446.5
(22) Date of filing: 22.09.2008
(51) Int. Cl.: C12Q 1/68

(54) **DIAGNOSTICS OF B-CELL LYMPHOMA**
DIAGNOSE VON B-ZELL-LYMPHOM
DIAGNOSTIC D'UN LYMPHOME B

(30) Priority: 20.09.2007 US 973871 P
(43) Date of publication of application: 02.06.2010
(73) Proprietor: ValiPharma, London EC1N 8SS (GB)
(72) Inventor: KROHN, Kai, FI-36450 Salmentaka (FI)
(74) Representative: Potter Clarkson LLP
(86) International application number: PCT/FI2008/050523
(87) International publication number: WO 2009/037385

(56) References cited:
- WO-A1-03/066898
- WO-A1-2005/038057
- US-A1- 2003 219 760
- KARENKO L ET AL.: "Primary cutaneous T-cell lymphomas show a deletion or translocation affecting NAV3, the human UNC-53 homologue", CANCER RESEARCH, vol. 65, no. 18, 2005, pages 8101-8110,7692, XP002662100,
- LESTOU V S ET AL.: "Multicolour fluorescence in situ hybridization analysis of t(14;18)-positive follicular lymphoma and correlation with gene expression data and clinical outcome", BRITISH JOURNAL OF HAEMATOLOGY, vol. 122, 2003, pages 745-759, XP002662101,
- HALLERMANN C ET AL.: "Chromosomal aberration patterns differ in subtypes of primary cutaneous B cell lymphomas", JOURNAL OF INVESTIGATIVE DERMATOLOGY, vol. 122, 2004, pages 1495-1502, XP002662102,
- DUMMER R ET AL.: "Perspektiven der Forschung bei kutanen Lymphomen", JDDG, vol. 4, June 2006 (2006-06), pages 492-495, XP002662103,
- SIPILA L ET AL: "376 POSTER NAV3-anovel cancer biomarker", EUROPEAN JOURNAL OF CANCER. SUPPLEMENT, vol. 6, no. 12, 1 October 2008 (2008-10-01), page 118, XP025534440, PERGAMON, OXFORD, GB ISSN: 1359-6349, DOI: 10.1016/S1359-6349(08)72310-3 [retrieved on 2008-10-01]
- AL-ASSAR, O. ET AL.: 'Transformed diffuse large B-cell lymphomas with gains of the discontinuous 12q12-14 amplicon display concurrent deregulation of CDK2, CDK4 and GADD153 genes.' BRITISH JOURNAL OF HAEMATOLOGY vol. 133, no. 6, 2006, pages 612 - 621, XP008131335
- REES, K. ET AL.: 'Genetic events associated with transformation of non-Hodgkin's lymphoma.' BLOOD vol. 98, no. 11, 2001, page 576A, XP008131479
- MARTINEZ-CLIMENT, JA.: 'Transformation of follicular lymphoma to diffuse large cell lymphoma is associated with a heterogeneous set of DNA copy number and gene expression alterations.' BLOOD vol. 101, no. 8, 2003, pages 3109 - 3117, XP002492754
- LOSSOS IS. ET AL.: 'Prediction of survival in diffuse large-B-cell lymphoma based on the expression of six genes.' THE NEW ENGLAND JOURNAL OF MEDICINE vol. 350, no. 18, 2004, pages 1828 - 1837, XP009045640

## Description

### FIELD OF THE INVENTION

The present invention relates to the fields of genetics and oncology and provides methods and means for diagnosing and monitoring of patients having B-cell lymphomas, such methods and means allowing an early diagnosis of the B-cell lymphoma. Specifically, the present invention relates to a novel method and a biomarker for diagnosing B-cell lymphomas and for differentiating the B-cell lymphomas into prognostic groups of indolent and aggressive B-cell lymphomas.

### BACKGROUND OF THE INVENTION

Current concept of cancer development includes the development of chromosomal instability, aneuploidy and a series of acquired genetic aberrations affecting genes important for the growth and survival of the cell. Stem cells have been suggested to play a critical role in cancer development, and epigenetic regulation is relevant for pluripotency-associated gene expression. Also single-gene aberrations may have influence on the whole pathogenesis process, and furthermore, may be useful as cancer biomarkers. During the past years acquired genetic aberrations have been found in various cancers (e.g. HER/neu in breast cancer) and molecular cytogenetic assays based on these genes have reached the markets and are in routine cancer diagnostic use in clinical laboratories.

Lymphomas are cancers of lymphoid tissue. They comprise a group of heterogenous cancers, divided into non-Hodgkin's lymphomas (NHL) and Hodgkin's lymphomas (HL). There are more than 40 subgroups, depending on the type and maturity of the underlying malignant lymphoid cell. NHL usually originates in lymphoid tissues and can be classified as either B-cell or T-cell non-Hodgkin lymphoma. Most (i.e., 80-90%) NHLs are of B-cell origin. B-cell non-Hodgkin lymphomas include Burkitt lymphoma, diffuse large B-cell lymphoma, follicular lymphoma, immunoblastic large cell lymphoma, precursor B-lymphoblastic lymphoma, and mantle cell lymphoma. T-cell non-Hodgkin lymphomas include mycosis fungoides, anaplastic large cell lymphoma, and precursor T-lymphoblastic lymphoma. Lymphomas related to lymphoproliferative disorders following bone marrow or stem cell transplantation are usually B-cell non-Hodgkin lymphomas.

The prognosis of NHL depends on the histologic type, stage, and treatment. Based on these variables, the NHLs can be divided into two prognostic groups: the indolent lymphomas and the aggressive lymphomas. Indolent NHL types have a relatively good prognosis but they are not curable in advanced clinical stages. Most of the indolent types are nodular (or follicular) in morphology. Follicular lymphoma (FL) is by far the most common of the indolent NHLs and represents almost 25% of all new cases of NHL. The aggressive type of NHL (e.g. diffuse large B-cell lymphoma (DLBCL)) has a shorter life expectancy. With modern treatment of patients with NHL, overall survival at 5 years is approximately 50% to 60%. Of patients with aggressive NHL, 30% to 60% can be cured but the vast majority of relapses occur in the first 2 years after therapy.

The incidence of various lymphomas is continuously increasing and is currently around 363 000 new cases annually worldwide.

In the treatment of lymphomas, an early diagnosis is crucial, because the disease is prone to relapse in later stages. In the diagnosis of lymphoma, a biopsy sample is obtained from the lymph node and a histopathological analysis is performed. The type of lymphoma can be identified by the physical appearance of the cancer cells under the microscope, or by using markers that identify special molecules on the lymphoma cells. Follicular lymphoma grading is based on the average number of large transformed cells in 10 neoplastic follicles at x10-40 high-power field examination. The reproducibility of follicular lymphoma grading is dependent upon observer experience; therefore, significant variations occur.

Currently, there are no molecular biologic biomarkers available in the clinic in order to identify patients with NHL as early as possible, or to identify patients in whom the malignant cell clones persist despite clinically efficient therapy.

Chromosomal aberrations are frequently seen in lymphomas and quite often such aberrations occur in the 12q region (Bea et al., 1996, Benz et al., 1996, Horsman et al, 2001, Hernandez et al., 2001, Lestou et al., 2003, Chui et al., 2003, Hallerman et al., 2004). The most common chromosomal abnormality associated with NHL is the t(14;18)(q32;q21) translocation that is found in 85% of follicular lymphomas and 25-30% of intermediate-grade NHLs. This translocation results in the juxtaposition of the *bcl*-2 apoptotic inhibitor oncogene at chromosome band 18q21 to the heavy-chain region of the immunoglobulin (Ig) locus within chromosome band 14q32, resulting in its overexpression. The t(11;14)(q13;q32) translocation results in overexpression of *bcl*-1 (cyclin-D1/*PRAD1*), a cell cycle control gene on chromosome band 11q13, and is diagnostic of mantle cell lymphoma. In follicular lymphomas an increased frequency of chromosomal gains involving chromosome 12 among others, have been revealed by MFISH analysis (Benz *et al.,* 1996, Horsman *et al.,* 2001, Lestou *et al.,* 2003), and increased gene expression of chromosome bands with gains, including SAS gene in 12q13-q14 was observed (Lestou *et al.,* 2003). It is considered that translocation events are primarily responsible for FL disease initiation, whereas the unbalanced chromosomal gains and losses (which also mirror the gene expression patterns) characterize clonal evolution and disease progression. Accordingly, del(17p) and gain/amplification of chromosome 12 have been shown to be correlated with an adverse clinical outcome or transformation to diffuse large cell lymphoma (DLCL) (Martinez-Climent *et al.,* 2003, Höglund *et al.,* 2004). So far it is unknown, however, which gene or genes are affected by the aforementioned chromosomal changes in chromosome 12.

Recently, a novel putative tumor suppressor gene NAV3 was shown to be deleted/translocated in most of the common cutaneous T-cell lymphoma types (CTCL) (Karenko et al., 2005, WO03066898). Within B-cell lymphomas, aberrations in Bcl-2, CD20, PAX-5, and BCL-6 have been described but they have not reached wide clinical applications.

Thus, novel biomarkers for providing more effective and early diagnosis of B-cell lymphomas as well as identifying B-cell lymphomas susceptible to targeted therapies are warranted. Novel biomarkers for differentiating lymphomas are also needed.

### BRIEF DESCRIPTION OF THE INVENTION

The invention is defined in the accompanying claims.

The object of the invention is thus to provide novel methods and means for diagnosing and monitoring of patients having symptoms of B-cell lymphomas, such methods and means allowing an early and specific diagnosis of the B-cell lymphoma and identifying B-cell lymphomas susceptible to targeted therapies.

Another object of the invention is to provide novel methods and means allowing early identification of patients with an increased risk to develop aggressive lymphoma and thus enabling efficient cancer prevention.

A further object of the invention is to provide novel methods and means for the development of new guidelines for follow-up of therapeutic interventions. Also described herein but not claimed is the development of new treatment modalities for B-cell lymphomas, such methods and means prolonging the remission stage of the disease and introducing new possibilities for combating the disease and for the recovery of the patient.

Still another object of the invention is to provide novel methods and means for differentiating B-cell lymphomas into prognostic groups of indolent and aggressive forms.

The present invention relates to a method, which is characterized by detecting genetic aberrations of NAV3 gene in a biological sample, specifically changes in NAV3 gene copy number, the presence of aberrations indicating B-cell lymphoma.

The present invention further relates to a method of detecting genetic aberrations of NAV3 gene, preferably loss or gain, the loss or gain of NAV3 potentiating differentiation of the B-cell lymphomas into prognostic groups of indolent and aggressive lymphomas.

In one preferred embodiment of invention the genetic aberrations are determined by fluorescence *in situ* hybridization (FISH).

In another preferred embodiment of invention the B-cell lymphoma is a follicular lymphoma or a diffuse large B-cell lymphoma.

The present invention also relates to a use of *NAV3* gene for diagnosis or therapy of B-cell lymphomas.

The present invention further relates to a use of *NAV3* gene as a biomarker for B-cell lymphomas, preferably NAV3 gene is used as a marker of B-cell lymphoma malignancy.

The present invention opens new possibilities in the advancement of therapies for B-cell lymphomas. Categorizing patients in the two subgroups of indolent and aggressive forms of lymphoma will allow the clinician in charge of the treatment of the patient to select individual therapeutic modalities, most effective in the given patient.

With the invent of novel biomarkers, such as chromosomal and genetic differences within a group of lymphomas having the same original diagnosis, the list of different type of lymphomas will increase and will in the future provide means and basis for more specific therapy (as characterized by the term "personalized medicine".

### BRIEF DESCRIPTION OF THE DRAWINGS

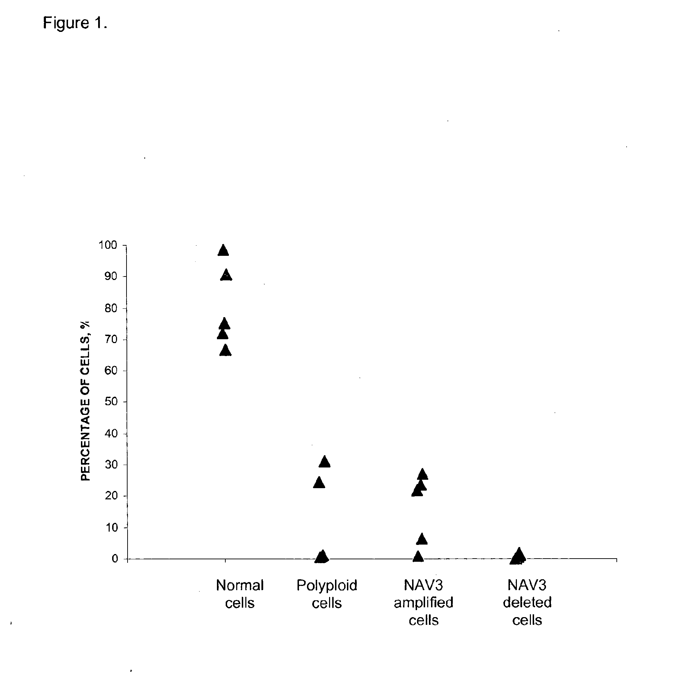

In the following the invention will be described in greater detail by means of preferred embodiments with reference to the attached drawings, in which
Figure 1 shows the result of *NAV3*-specific FISH with follicular lymphoma. Each triangle represents a separate case. Five cases were analyzed and 200 cells were counted from each case. Cells were grouped as normal, polyploid cells, *NAV3* deleted cells or *NAV3* amplified cells. Results are shown as percentage of each cell type.

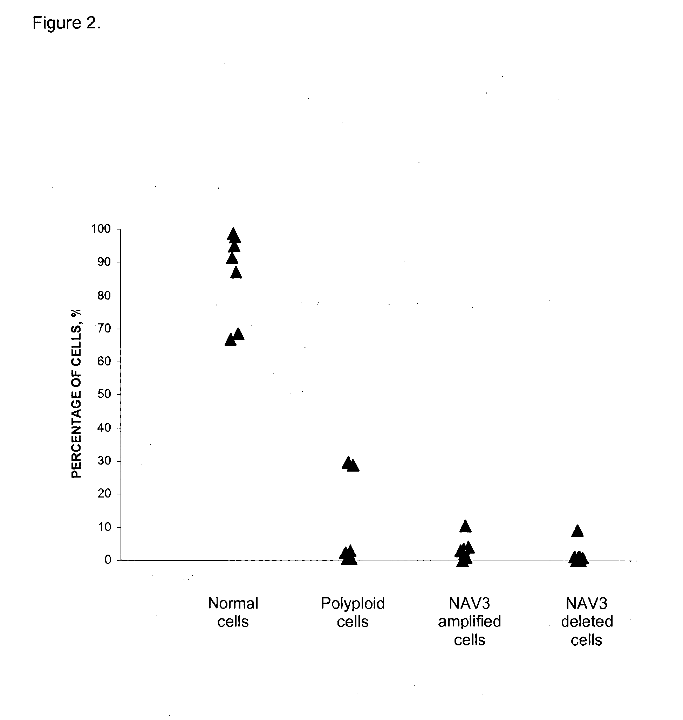

Figure 2 shows NAV3-specific FISH with diffuse large B-cell lymphoma samples. Each triangle represents a separate case. Seven cases were analyzed and 200 cells were counted from each case. Cells were grouped as normal, polyploid cells, *NAV3* deleted cells or *NAV3* amplified cells. Results are shown as percentage of each cell type.

### DETAILED DESCRIPTION OF THE INVENTION

The present invention is based on a method for detecting genetic aberrations in NAV3 gene, wherein the aberrations are detected by copy number deviations from normal.

As used herein the expression "genetic aberration" refers to deletions (loss) or amplifications (gain) of the NAV3 gene, which can be detected as a change in the copy number of the gene.

As used herein the expression "deletion" refers to the absence of a NAV3 gene fragment, gene or chromosomal fragment containing the gene. In a preferred analysis method deletion means less than two copies of NAV3 signal.

As used herein the expression "amplification" refers to gain of genetic material such as a gene fragment, gene or chromosomal fragment containing the gene. In a preferred analysis method amplification means more than two copies of NAV3 signal.

As used herein the expression "polyploid cell" refers to cells having more than two copies of chromosome centromere.

As used herein the expression "B-cell lymphoma" refers to a non-Hodgkin's lymphomas caused by malignant (cancerous) B-cell lymphocytes.

NAV3 (neuronal navigator, also called POMFIL1) is a spliced gene (40 exons) located in chromosome 12q21 and expressed in brain tissue, activated T-cells, placenta, colon and in certain cancer cell lines (Coy et al. 2002; Maes et al., 2002; Karenko et al. 2005). The expression of NAV3 is strongly reduced in 40% of primary tumors of neural or glial origin and, on the other hand, upregulated after brain injury (Coy et al., 2002).

The amino acid sequence of NAV3 is well conserved among different species, which indicates that NAV3 has an important task in cellular processes. As predicted from the amino acid sequence, NAV3 may have a role in cell signaling or tumor suppression. It also shows the properties of a helicase (enzyme which unwinds the DNA helical structure); helicases have a role in the maintenance of the stability of chromosomes, and their deficiency could cause a hyperrecombination phenotype with deletion mutants, and also loss of heterozygosity and increase in sister chromatid exchanges.

In the present invention, changes in NAV3 copy number were studied in a series of samples from the two most frequent B-cell lymphomas by fluorescence *in situ* hybridization (FISH) utilizing locus-specific probes. In addition to NAV3 deletion, number of cells with polyploidy and NAV3 amplification was recorded. NAV3 amplification was found in follicular lymphoma (Figure 1), but both amplification as well as deletion was found in the more aggressive form of B-cell lymphoma, the diffuse large cell lymphoma (Figure 2). Thus, genetic aberrations of *NAV3* gene characterized by loss or gain of NAV3 appear as a marker of malignancy in lymphomas originating from B-lymphocytes (B-cells).

According to one preferred embodiment of the present invention, the presence or absence of the *NAV3* gene can be detected from a biological sample by any known detection method suitable for detecting a gene expression or copy number, i.e. methods based on detecting the copy number of the gene (or DNA) and/or those based on detecting the gene expression products (mRNA or protein). Such methods are easily recognized by those skilled in the art and include conventional polymerase chain reaction (PCR)-methods, RT-PCR, *in situ* hybridisations, such as FISH, array-CGH, mRNA *in situ* hybridisation, high-density single nucleotide polymorphism (SNP) arrays, Northern analysis, Southern and Western analyses, immunohistochemistry, and other immunoassays, such as ELISA. Preferable methods are those suitable for use in routine clinical laboratories. Most preferably the change in NAV3 copy number is detected by FISH.

In the method of the invention, the biological sample can be any suitable tissue sample, such as biopsy from the lymph node or bone marrow. The biological sample can be, if necessary, pretreated in a suitable manner known to those skilled in the art.

Also described herein but not claimed is the therapeutic restoration of the normal function of the *NAV3* gene. This may be reached by enhancing the expression of functionally homologous genes, by introducing an intact *NAV3* gene or by using an altered form of the *NAV3* gene or antisense oligonucleotide against the *NAV3* in any technique presently available for gene therapy to prevent the progression of a proliferating disease. In particular, tumor cell growth may be slowed down or even stopped by such therapy. Such techniques include the *ex vivo* and *in situ* therapy methods, the former comprising transducing or transfecting an intact or altered *NAV3* gene (or its functional domains) in a recombinant or peptide form or as antisense oligonucleotides or in a vector to the patient, and the latter comprising inserting the altered gene or oligonucleotide into a carrier, which is then introduced into the patient. Depending on the disease to be treated, a transient cure or a permanent cure may be achieved. Alternatively, monoclonal or humanized antibodies or peptides binding to the NAV3 protein or to the fusion gene generated as a result of the translocation, can be used to suppress the function of the altered NAV3 protein and thus tumor cell growth may be slowed down or even stopped. Antibodies against NAV3 could also be used to carry other agents, such as cytotoxic substances, to the cancer cells over-expressing the *NAV3* gene. Such agents could then be used to kill specifically the cancer cells.

The following examples are given for further illustration of the invention.

### EXAMPLE 1

### NAV3-SPECIFIC FISH-ANALYSIS OF B-CELL LYMPHOMAS

### SAMPLES

Samples for the FISH assay were prepared from 12 randomly selected B cell lymphoma cases. Five cases represented follicular lymphoma (FL) and seven cases diffuse large B-cell lymphoma (DLBCL). All tissue sampies were so called touch imprint samples, prepared from fresh biopsy material by pressing the biopsy gently against a Super Frost Plus slide so that cells were left on the slide. Touch imprint slides were stored at -70°C.

### PROBE LABELING

Two bacterial artificial chromosome (BAC) clones specific to *NAV3* DNA (RP11-36P3 and RP11-136F16; Research Genetics Inc., Huntsville, AL, USA) were labeled with Alexa594-5-dUTP (Invitrogen) and the chromosome 12 centromere probe (pA12H8; American Type Cell Culture) was labeled with Alexa488-5-dUTP (Invitrogen) using nick translation (Hyytinen et al. 1994). 50-75 ng of each labeled BAC and 30 ng of centromere probe were mixed together with 1 µg of human COT1 DNA (Invitrogen) and precipitated with sodium acetate and ethanol. Precipitated probe mix was diluted into 10 µl of hybridization buffer (15% w/v dextran sulphate, 70% formamide in 2x SSC, pH 7.0).

### FLUORESCENCE IN SITU HYBRIDISATION

Slides were fixed with 4% paraformaldehyde in PBS for 1 minute on ice. After PBS washes, slides were digested enzymatically with proteinase K (Sigma; 0.66µg/ml in 20 mM Tris-HCl, pH 7.5, 2 mM CaCl₂) at +37°C for 6 minutes. After dehydration and air drying probe mix was pipetted on slides and slides were denatured for 5 min at +75°C on a hot plate. Hybridisation was carried out for 48 hr at +37°C. Slides were washed three times with 1.5 M Urea, 0.1 x SSC at +47°C for 10 minutes, once with 0.1 x SSC for 10 minutes at +47°C, followed by three washes with PBS, 0.1 % NP-40 at room temperature. Finally, slides were rinsed with distilled water, air dried and mounted in Vectashield Mounting Medium with 4',6-diamino-2 phenylindole dihydrochloride (DAPI; Vector).

### ANALYSIS AND RESULTS

FISH results were evaluated using Olympus BX61 microscope (Tokyo, Japan) equipped with a 60X oil immersion objective and a triple bandpass filter for simultaneous detection of Alexa488, Alexa594 and DAPI (Chroma Technology Corp., Brattleboro, VT, USA). 200 cells were analysed from each case and the cells were grouped as normal if having two labels for chromosome 12 centromere and two for the *NAV3.* Polyploid cells had three or more centromere labels. *NAV3* deletion was defined when the number of centromere labels was higher than the number of *NAV3* labels and *NAV3* amplification was defined when the number of *NAV3* labels was higher than centromere labels. The analyses were done blinded to the diagnosis or sample identity by two independent analyzers. 3/5 studied follicular lymphoma cases (60%) showed clear NAV3 amplification. No NAV3 deletion was detected (Figure 1). In case of diffuse large B-cell lymphomas 1/7 (14%) sample showed NAV3 amplification and 1/7 (14%) sample showed NAV3 deletion (Figure 2).

## Claims

1. A method for the diagnosis of B-cell lymphoma, **characterized by** detecting genetic aberrations of NAV3 gene in a biological sample the presence of aberrations indicating B-cell lymphoma.

2. A method according to claim 1, **characterized in that** genetic aberrations are changes in NAV3 gene copy number.

3. A method according to claim 2, wherein the loss or gain of NAV3 enables differentiation of B-cell lymphomas into prognostic groups.

4. A method according to claim 3, wherein the prognostic groups are indolent and aggressive form of B-cell lymphomas.

5. A method according to any preceding claim, **characterized in that** genetic aberrations are determined by fluorescence *in situ* hybridization (FISH).

6. A method according to any preceding claim, **characterized in that** the B-cell lymphoma is a follicular lymphoma.

7. A method according to any preceding claim, **characterized in that** the B-cell lymphoma is a diffuse large B-cell lymphoma.

8. Use of NAV3 gene as a biomarker for B-cell lymphomas.

9. Use of claim 8, **characterized in that** loss or gain of *NAV3* gene is a marker of B-cell lymphoma malignancy.

## Patentansprüche

1. Verfahren zur Diagnose von B-Zell-Lymphom, **gekennzeichnet durch** das Detektieren genetischer Aberrationen des NAV3-Gens in einer biologischen Probe, wobei das Auftreten von Aberrationen indikativ für B-Zell-Lymphom ist.

2. Verfahren nach Anspruch 1, **dadurch gekennzeichnet, dass** die genetischen Aberrationen Veränderungen in der Kopienzahl des NAV3-Gens sind.

3. Verfahren nach Anspruch 2, wobei der Verlust oder die Zunahme von NAV3 eine Differenzierung von B-Zell-Lymphomen in prognostische Gruppen ermöglicht.

4. Verfahren nach Anspruch 3, wobei die prognostischen Gruppen indolente und aggressive Form von B-Zell-Lymphomen sind.

5. Verfahren nach einem der vorhergehenden Ansprüche, **dadurch gekennzeichnet, dass** die genetischen Aberrationen mittels Fluoreszenz-*in-situ*-Hybridisierung (FISH) bestimmt werden.

6. Verfahren nach einem der vorhergehenden Ansprüche, **dadurch gekennzeichnet, dass** das B-Zell-Lymphom ein follikuläres Lymphom ist.

7. Verfahren nach einem der vorhergehenden Ansprüche, **dadurch gekennzeichnet, dass** das B-Zell-Lymphom ein diffuses großzelliges B-Zell-Lymphom ist.

8. Verwendung von NAV3-Gen als Biomarker für B-Zell-Lymphome.

9. Verwendung nach Anspruch 8, **dadurch gekennzeichnet, dass** der Verlust oder die Zunahme von NAV3-Gen ein Marker für die Malignität von B-Zell-Lymphom ist.

## Revendications

1. Procédé de diagnostic d'un lymphome à cellules B, **caractérisé par** la détection d'aberrations génétiques du gène NAV3 dans un échantillon biologique, la présence d'aberrations indiquant un lymphome à cellules B.

2. Procédé selon la revendication 1, **caractérisé en ce que** les aberrations génétiques sont des changements dans le nombre de copies du gène NAV3.

3. Procédé selon la revendication 2, dans lequel la perte ou le gain de NAV3 permet une différenciation de lymphomes à cellules B dans des groupes pronostiques.

4. Procédé selon la revendication 3, dans lequel les groupes pronostiques sont une forme indolente et agressive de lymphomes à cellules B.

5. Procédé selon l'une quelconque des revendications précédentes, **caractérisé en ce que** les aberrations génétiques sont déterminées par hybridation *in situ* en fluorescence (FISH).

6. Procédé selon l'une quelconque des revendications précédentes, **caractérisé en ce que** le lymphome à cellules B est un lymphome folliculaire.

7. Procédé selon l'une quelconque des revendications précédentes, **caractérisé en ce que** le lymphome à cellules B est un lymphome diffus à grandes cellules B.

8. Utilisation du gène NAV3 comme biomarqueur pour des lymphomes à cellules B.

9. Utilisation selon la revendication 8, **caractérisée en ce que** la perte ou le gain du gène NAV3 est un marqueur de malignité d'un lymphome à cellules B.
